(19) Europäisches Patentamt
European Patent Office
Office européen des brevets

(11) Veröffentlichungsnummer : **0 204 952**
**B1**

(12) # EUROPÄISCHE PATENTSCHRIFT

(45) Veröffentlichungstag der Patentschrift : **28.09.88**

(51) Int. Cl.[4] : **C 07 C 49/637**, C 07 C 45/66, A 23 L 1/226, A 61 K 7/46

(21) Anmeldenummer : **86106127.3**

(22) Anmeldetag : **05.05.86**

(54) **Verfahren zur Herstellung von 4,4,7-Trimethyl-3,4,7,8,-tetrahydro-2(6H)-naphthalin-on in gereiniger Form oder im Gemisch mit 3,5,5-Trimethyl-4-butenyliden-cyclo-hex-2-en-1-onen, dabei erhaltene Produkte und Verwendung derselben als Riech- und Aromastoffe.**

(30) Priorität : **10.05.85 DE 3516931**

(43) Veröffentlichungstag der Anmeldung : **17.12.86 Patentblatt 86/51**

(45) Bekanntmachung des Hinweises auf die Patenterteilung : **28.09.88 Patentblatt 88/39**

(84) Benannte Vertragsstaaten : **CH FR GB LI NL SE**

(56) Entgegenhaltungen :
**DE-A- 1 804 711**
**GB-A- 1 071 365**
**JOURNAL OF THE AMERICAN CHEMICAL SOCIETY, Band 75, Nr. 15, 5. August 1953; M. SULZBACHER et al.: "The dehydration of 4-hydroxy-2,2,4,5,5-pentamethyltetrahydrofuran (Acetylene studies, Part VI)"**

(73) Patentinhaber : **Dragoco Gerberding & Co. GmbH**
**Dragocostrasse 1**
**D-3450 Holzminden (DE)**

(72) Erfinder : **Brunke, Ernst-Joachim, Dr.**
**Holbeinplatz 6**
**D-3450 Holzminden (DE)**
Erfinder : **Tumbrink, Ludwig, Dr.**
**Wilhelm-Raabe-Strasse 22**
**D-3470 Höxter 1 (DE)**

(74) Vertreter : **Patentanwälte Müller-Boré, Deufel, Schön, Hertel, Lewald, Otto**
**Isartorplatz 6**
**D-8000 München 2 (DE)**

**EP 0 204 952 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die zur Aromatisierung von Nahrungs- und Genußmittel sowie Tabakwaren eingesetzten Aromen und die in der Parfümerie- und Kosmetikaindustrie verwendeten Riechstoffe werden zunehmend mehr durch chromatographische und spektroskopische Methoden aufgespürt, worauf ihre Konstitution ermittelt wird und bei Bedarf die synthetische Herstellung erfolgt. Einen breiten Raum nehmen in diesen Untersuchungen die aromagebenden Inhaltstoffe der verschiedenen Tabakarten ein und eine Übersicht über die große Zahl solcher Tabakinhaltsstoffe findet sich in C.R. Enzell, I. Wahlberg, A.J. Aasen, Fortschritte der Chemie organischer Naturstoffe, Bd. 34, Springer-Verlag, Wien und New York (1977), S. 1-79. Eine sensorisch wichtige Gruppe dieser natürlichen Tabakaromastoffe sind Carotinoid-Abbauprodukte und innerhalb dieser Gruppe kommt dem von A.J. Aasen, B. Kimland und C.R. Enzell (Acta Chem. Scand. 25, 1481-1482 (1971)) aus Tabak isolierten 3-Oxo-α-jonol (Verb. (1) im unten angegebenen Formelschema) eine Schlüsselstellung zu, da es als Vorstufe zu weiteren Abbauprodukten gelten kann (vgl. obige Druckschrift Enzell et al). Derartige Folgeprodukte sind unter anderem die aus (1) resultierenden, mit (2) bis (6) bezeichneten Dehydratisierungsprodukte, die ebenfalls im Tabak nachgewiesen worden sind (vgl. A.J. Aasen, B. Kimland, S.-O. Almquist und C.R. Enzell, Acta Chem. Scand. 26, 2573-2576 (1972), im folgenden als Referenz-Druckschrift A bezeichnet) und für die von den genannten Autoren die Bezeichnung Megastigmatrienone vorgeschlagen wurde.

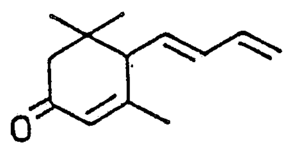

2

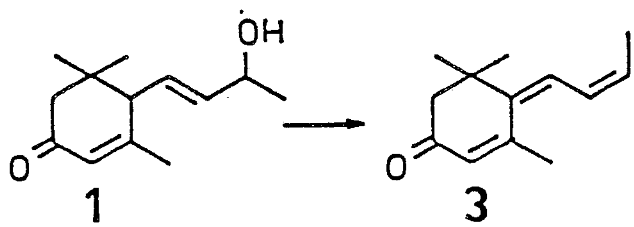

1

3

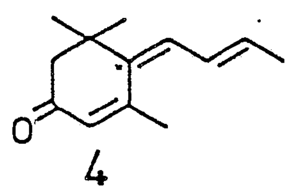

4

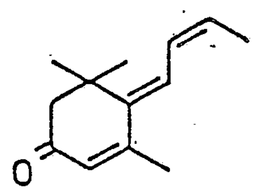

5

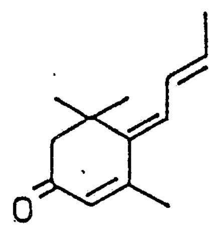

6

Die Megastigmatrienone (3) bis (6) sind auch im etherischen Öl von « Kudzu » (Puerania lobata) von S. Shibata et al. (J. Agric. Biol. Chem., 42, 195-197 (1978)) und als Bestandteil der flüchtigen Komponenten von «Kukoshi »-Beeren (Lycium chinense M.) von A. Sannai et al. (J. Agric. Biol. Chem., 47, 2397-2399 (1983)) gefunden worden.

Die erste Synthese für die Megastigmatrienone (3) bis (6) wurde bereits im Jahr 1963 beschrieben (R.L. Rowland, US-PS 3 211 157), d. h. lange vor dem Bekanntwerden dieser Substanzen als Tabakinhaltsstoffe (vgl. Referenz-Druckschrift A). Zur Durchführung der Synthese wurde Dehydro-α-jonon (7) mit Natriumborhydrid zum Alkohol (8) reduziert, der mit 2 N-Schwefelsäure zu (9) umgelagert wurde. Braunstein-Oxidation des Allylalkohols (9) ergab die Megastigmatrienone (3) bis (6).

(Siehe Schema Seite 3 f.)

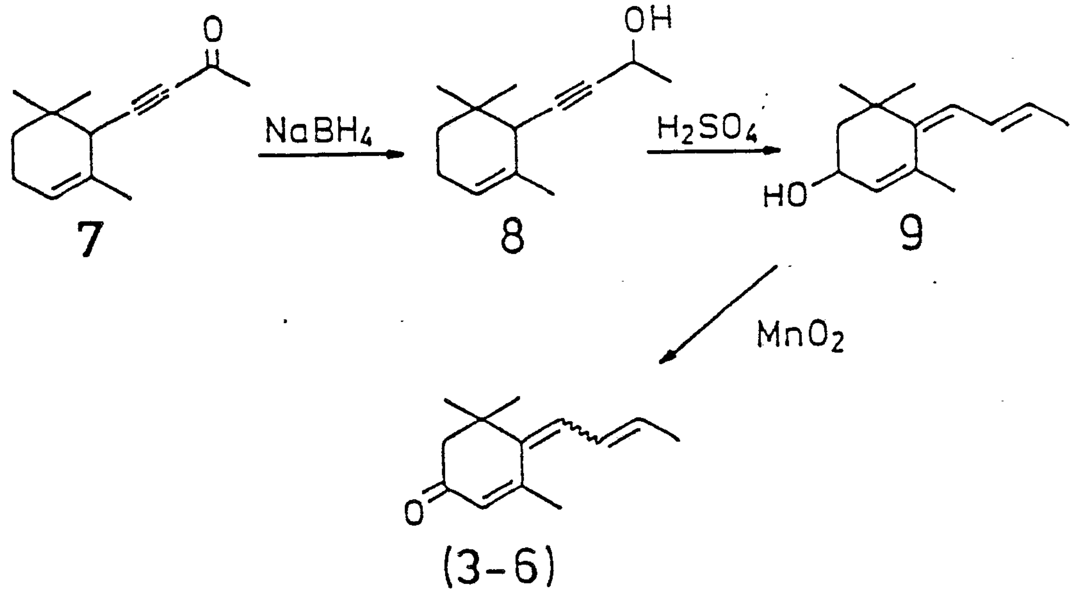


7 8 9

(3-6)

E. Demole und P. Enggist (Helv. Chim. Acta, 57, 2087-2089 (1974)) beschreiben eine Synthese ausgehend von dem aus Oxo-isophoron erhaltenen Ketoketal (10), das mit dem Dianion des But-3-in-2-ols und anschließend mit Lithium-aluminiumhydrid umgesetzt wurde und ein Gemisch von Megastigmatrienonen (3) bis (6) mit verschiedenen Hydroxy-ketonen vom Megastigman-Typ (11) bis (14) ergab.

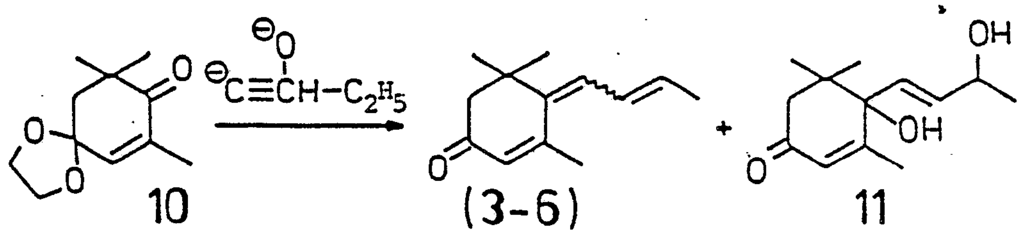


10 (3-6) + 11

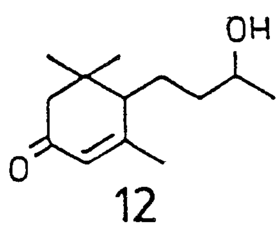

12

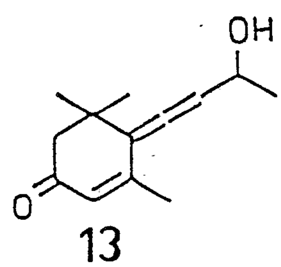

13

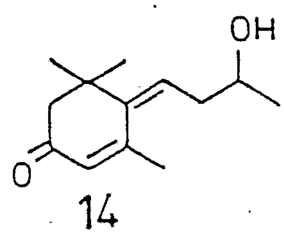

14

Ausgehend von Dimedon (15) erhielten S. Torii, T. Inokuchi und H. Ogawa (Bull. Chem. Soc. Japan, 52, 1233-1234 (1979)) durch Aldolkondensation des Enolats mit Crotonaldehyd und anschließende Dehydratisierung die enolisierten Diketone (16), deren Umsetzung mit Methyllithium zu den Megastigmatrienonen (3) bis (6) führte.

B.M. Trost und J.L. Stanton (J. Amer. Chem. Soc., 97, 4018-4025 (1975)) reduzierten das 3,4-Dehydro-ß-jonon (17) und erhielten durch Dehydratisierung und Sulfenylierung die Verbindung (18) die über eine Sulfenat-Sulfoxid- Umlagerung ind die Megastigmatrienone (3) bis (6) umgewandelt wurde.

O. Takazwawa, H. Tamura, K. Kogami und K. Hayashi (Bull. Chem. Soc. Japan, 55, 1907-1911 (1982)) beschreiben eine Synthese der Megastigmatrienone (3) bis (6) ausgehend von Isophoron (19), dessen Trimethylsilyl-enolether (20a) mit Crotonaldehyd kondensiert wurde. Das so erhaltene Oxo-damascol (20b) wurde zum Gemisch der Megastigmtrienone (3) bis (6) dehydratisiert.

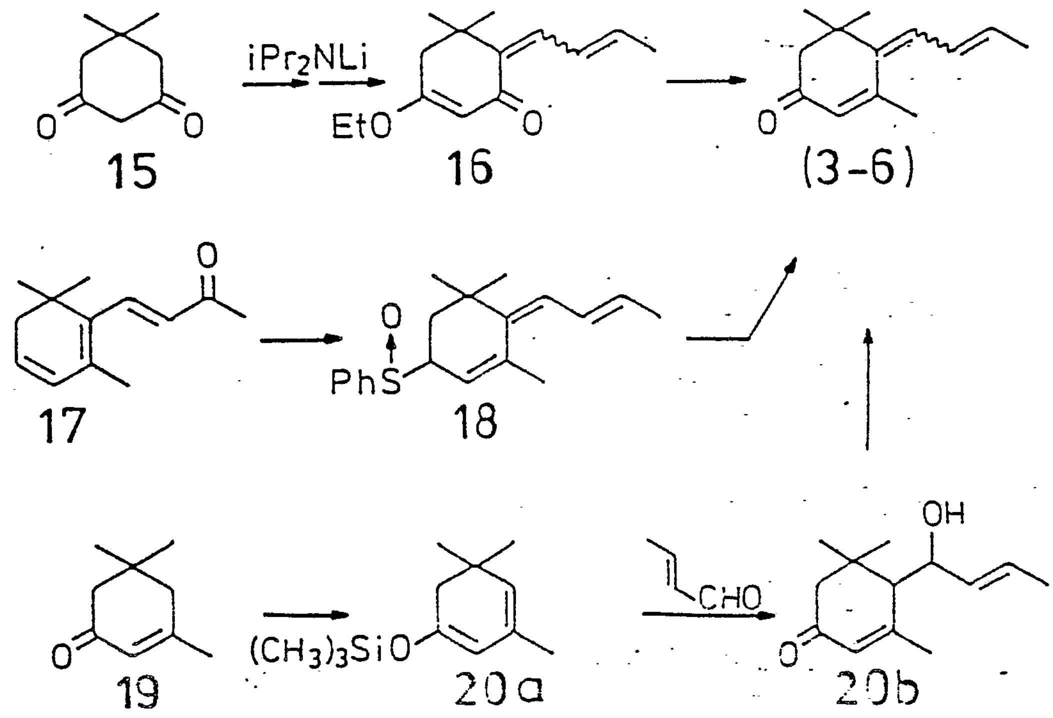


15 16 (3-6)

17 18

19 20a 20b

Die säurekatalysierte Dehydratisierung von 3-Oxo-α-jonol (1) ist, soweit bekannt, bisher nur einmal in der Literatur beschrieben worden. Aasen, Kimland, Almquist und Enzell (vgl. Referenz-Druckschrift A) erhitzten 3-Oxo-α-jonol (1) mit Kaliumhydrogensulfat (100 °C, ohne Lösunsgsmittel) oder mit para-Toluolsulfonsäure (Benzol, Rückfluß) 2 bis 3 h lang. Hierbei erhielten die Autoren stets ein Gemisch der isomeren Megastigmatrienone (2) bis (6), deren Isomerenverhältnis von ihnen gaschromatographisch zu

(2) : (3) : (4) : (5) : (6) = 10 : 1 : 10 : 1 : 10

bestimmt wurde.

Das aus (1) dargestellte Acetat soll gemäß Referenz-Druckschrift A unter den genannten Bedingungen zum gleichen Produktgemisch führen. Experimentelle Einzelheiten sind jedoch nicht angegeben worden.

Bei den bisher beschriebenen Synthesen der Megastigmatrienone (2) und (3) bis (6) findet sich über bicyclische Nebenprodukte nicht der geringste Hinweis, was besonders bemerkenswert für die ausführliche Arbeit von Enzell et al. (vgl. Referenz-Druckschrift A) ist, wonach bei der Dehydratisierung von 3-oxo-α-jonol (1) ausschließlich die monocyclischen Verbindungen (2) bis (6) erhalten werden. Auch bei der Dehydratisierung von α-jonol (F.B. Whitfield und G. Sugowdz, Aust. J. Chem. 35, 591-600 (1982)) wurden nur monocyclische Produkte erhalten.

Überraschenderweise wurde nunmehr gefunden, daß aus 3-Oxo-α-jonol (1) unter bestimmten Verfahrensbedingungen das 4,4,7-Trimethyl-3,4,7,8-tetrahydro-2(6H)-naphthalin-on (Verb. (21)) entsteht, das bisher nur einmal in der Literatur genannt wurde, und zwar von D.L. Roberts und W.A. Rohde (Tob. Sci., 16, 107-112 (1972), im folgenden als Referenz-Druckschrift B bezeichnet), die das Keton (21) als Bestandteil des Burley-Tabaks beschreiben. Die Autoren isolierten (21) durch Säulenchromatographie und Gaschromatographie. Eine Synthese von (21) ist, soweit bekannt, bisher nicht beschrieben worden.

Wie durch das folgende Reaktionschema veranschaulicht und weiter unten näher erläutert wird, können durch Dehydratisierung von (1) unter Verwendung der von Enzell et al. in der Referenz-Druckschrift A angegebenen Reagentien, jedoch unter inverser Reaktionsführung, Produktgemische aus Megastigmatrienonen (3) bis (6) und bis zu etwa 10 % des bicyclischen Ketons (21) erhalten werden.

Ein aus (1) auf übliche Weise darzustellender 3-Oxo-α-jonol-niedrigalkylester, z. B. das 3-Oxo-α-jonol-acetat (22) kann erfindungsgemäß ebenfalls in ein Gemisch aus (3) bis (6) und (21) umgewandelt werden, und zwar vorteilhafterweise unter Verwendung von Natriumacetat in polaren aprotischen Lösungsmitteln. Dabei wird die Bildung von (2) sehr stark unterdrückt. Der Anteil an bicyclischem Keton (21) kann durch Temperaturerhöhung gesteigert werden.

(Siehe Schema Seite 5 f.)

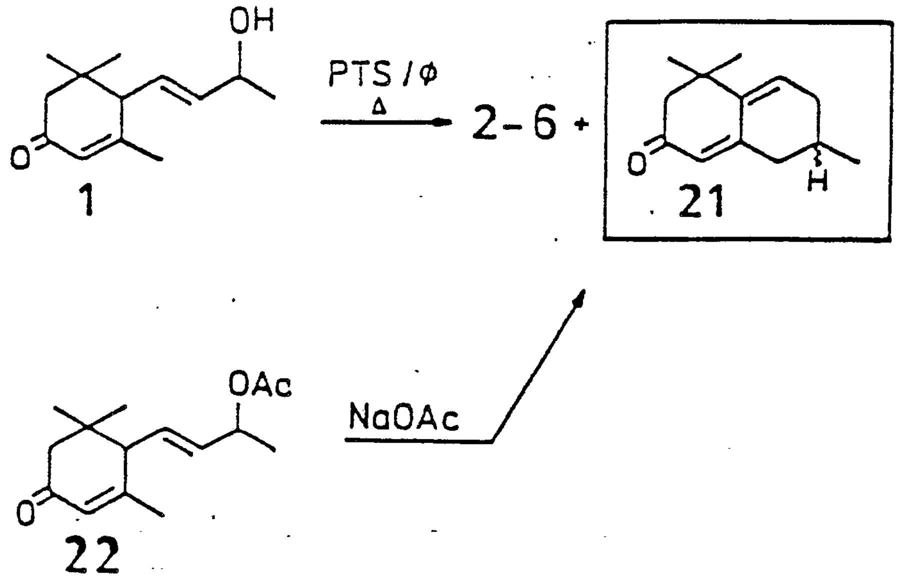


1 21 22

Die Erfindung wird durch die beigefügte Zeichnung näher veranschaulicht, in der darstellen

Fig. 1 : Gaschromatogramm des gemäß Stand der Technik erhaltenen Produktgemisches (vgl. Beispiel 1)

Fig. 2 : Gaschromatogramm des erfindungsgemäß aus 3-Oxo-α-jonol erhaltenen Produktgemisches (vgl. Beispiel 2)

Fig. 3 : Gaschromatogramm des erfindungsgemäß aus 3-Oxo-α-jonol-acetat erhaltenen Produktgemisches (vgl. Beispiel 3)

Fig. 4 : Gaschromatogramm eines erfindungsgemäß aus 3-Oxo-α-jonol-acetat bei vergleichsweise hohen Temperaturen erhaltenen Produktgemisches (vgl. Beispiel 4)

Fig. 5 bis 8 : IR-Spektrum, UV-Spektrum, $^1$H-NMR-Spektrum bzw. Massenspektrum des erfindungsgemäß hergestellten bicyclischen 4,4,7-Trimethyl-3,4,7,8-tetrahydro-2(6H)-naphthalin-ons (21) (vgl. Beispiel 3)

Die unten angegebenen Beispiele dienen der Erläuterung der Erfindung. So wurden in Beispiel 1 die in der Referenz-Druckschrift A angegebenen Reaktionsbedingungen zur Dehydratisierung von (1) wiederholt : Beim Erhitzen einer Mischung von (1) mit Kaliumhydrogensulfat auf 100 °C wurden die in der Referenz-Druckschrift A angegebenen Megastigmatrienone (2) bis (6) erhalten, allerdings in einem etwas abweichenden Produktverhältnis (Gaschromatogramm : Fig. 1). Ein weiterer Versuch zeigte, daß ein ähnliches Produktgemisch beim Kochen einer Lösung von (1) und para-Toluolsulfonsäure in Benzol unter Wasserabspaltung entstand.

Die von Enzell et al. in der Referenz-Druckschrift A für die Darstellung des Gemisches (2), (3) — (6) aus 3-Oxo-α-jonol (1) und dessen Acetat (22) angegebenen Reaktionsbedingungen ließen sich jedoch nur für das Carbinol (1), nicht aber für das Acetat (22) reproduzieren. Beim Erhitzen von (22) mit Kaliumhydrogensulfat auf 100 °C oder beim Kochen mit para-Toluol-sulfonsäure in Benzol wurde ausschließlich unverändertes Ausgangsmaterial zurückgewonnen, wie weitere Versuche zeigten.

Bei inverser Reaktionsführung gemäß Erfindung, d. h. bei Zutropfen der Lösung von 3-Oxo-α-jonol (1) zur siedenden Lösung der Säure, entstehen überraschenderweise neben den monocyclischen Ketonen (2) bis (6) auch größere Anteile des bicyclischen Ketons (21), wie Beispiel 2 zeigt. Gleichzeitig ist eine starke Abnahme des Ketons (2) festzustellen (Gaschromatogramm : Fig. 2). Es zeigte sich ferner, daß der Anteil an bicyclischem Keton (21) durch Temperaturerhöhung gesteigert werden kann, beispielsweise durch Arbeiten bei Siedetemperatur, z. B. durch Verwendung von siedendem Xylol als Lösungsmittel.

Die Reaktion kann auch unter Verwendung anderer Arylsulfonsäuren (z. B. Benzolsulfonsäure, p-Brombenzolsulfonsäure) oder saurer Ionenaustauscher in verschiedenen aromatischen Lösungsmitteln (z. B. Benzol, Toluol, Xylole, Chlorbenzol, Nitrobenzol) oder höhersiedenden Ethern )z. B. Diethylenglykoldimethylether, Ethylenglykoldibutylether) ausgeführt werden.

Aus den Beispielen 3 und 4 ergibt sich, daß das aus (1) auf übliche Weise gewonnene 3-Oxo-α-jonol-acetat (22) ebenfalls in ein Gemisch aus (3) bis (6) und (21) umgewandelt werden kann, was vorteilhafterweise unter Verwendung von Natriumacetat in polaren aprotischen Lösungsmitteln geschieht. Bei Verwendung von Natrium-acetat/Ethylenglykol (180 °C) (Beispiel 3) entsteht ein Produktgemisch das ca. 15 % (21) enthält (Gaschromatogramm : Fig. 3). Durch Erhöhung der Reaktionstemperatur, z. B. durch Verwendung höhersiedender Lösungsmittel, läßt sich der Anteil des bicyclischen Ketons (21) steigern. Bei Verwendung von Natriumacetat/Ethylenglykol (200 °C) (Beispiel 4) entsteht ein Produktgemisch, das ca. 70 % (21) enthält (Gaschromatogramm : Fig. 4). Unter analogen Reaktionsbedingungen lassen sich auch die Megastigmatrienone (3) bis (6) zu (21) cyclisieren.

Weitere geeignete Reagentien sind Hydroxide oder Niederalkansäuresalze anderer Alkali- oder Erdalkalimetalle, beispielsweise Natriumhydroxid, Natriumpropionat, Kaliumhydroxid, Kaliumacetat.

Ebenfalls geeignete Lösungsmittel sind höhersiedende Ether, z. B. Ethylenglykoldibutylether oder Diethylenglykoldimethylether, oder entsprechend hochsiedende Benzinfraktionen.

Man kann also ggfs. die neben 4,4,7-Trimethyl-3,4,7,8-Tetrahydro-2(6H)-naphthalin-on entstehenden 3,5,5-Trimethyl-4-butenyliden-cyclohex-2-en-1-one — vor oder Entfernen des Naphthalin-ons — durch Verlängern der Reaktion (5-10 h) in Gegenwart von Alkali- oder Erdalkalihydroxiden oder -acetaten bei erhöhten Temperaturen, vorzugsweise zwischen 150 und 300 °C, zu weiterem 4,4,7-Trimethyl-3,4,7,8-tetrahydro-2(6H)-naphthalin-on cyclisieren.

Beispiel 5 läßt erkennen, daß bei drastischer Temperaturerhöhung ohne Verwendung von Reagentien, z. B. bei der Pyrolyse des Acetats (22) (ca. 500 °C) ausschließlich das Megastigmatrienon (2) entsteht. Für die Bildung des bicyclischen Ketons (21) aus (1) oder (22) scheint somit das Vorliegen von Säuren oder Basen sowie eine in gewissem Umfang erhöhte Temperatur erforderlich zu sein.

Durch übliche Methoden, wie fraktionierte Destillation sowie präparative Gaschromatographie oder Säulenchromatographie, auch unter erhöhtem Druck (HPLC), kann das erfindungsgemäß synthetisierte Keton (21) weiter angereichert, bzw. in reiner Form gewonnen werden. Die spektroskopischen Daten des isolierten synthetischen Ketons (21) entsprechen weitgehend den in der Referenz-Druckschrift B angegebenen Daten des Naturstoffs, wie Tabelle 1 zeigt.

Tabelle 1 : Spektroskopische Daten von (21)

| | Naturstoff (gem. Ref. B) | | erfindungsgem. Syntheseprodukt | |
|---|---|---|---|---|
| IR, $\gamma$ ($cm^{-1}$) | 1660, 1622, 1596, 1300, 1250 | | 1665, 1625, 1595, 1300, 1250 | (Fig. 5) |
| UV, $\lambda_{max}$ ($\varepsilon'$) | 287 (90.3) | | 289 (57.5) | (Fig. 6) |
| $^1$H-NMR (solvent?) $\delta$[ppm] | 5.95 (1H) | ($CCl_4$) | 5.9 - 6.1 (1H) | (Fig. 7) |
| | 5.62 (1H) | | 5.53 (1H) | |
| | 2.20 (3H) | | 2.21 (3H) | |
| | 1.20 (3H) | | 1.18 (3H) | |
| | 1.15 (3H) | | 1.13 (3H) | |
| | 1.03 (3H, d) | | 1.04 (3H, d) | |
| MS, m/z | 190 ($M^+$) | | 190 ($M^+$) | (Fig. 8) |

Die Testung der olfaktorischen und sensorischen Eigenschaften von (21) und Gemischen von (21) mit (3) bis (6) ergab, daß das Megastigmatrienon (2) mit dekonjugiertem Doppelbindungssystem (Darstellung vgl. Beispiel 5) weniger wünschenswerte olfaktorische und sensorische Eigenschaften besitzt. Sein Vorhandensein in Eliminierungs-Produktgemischen des 3-Oxo-α-jonols (1) führt daher zu Beeinträchtigungen bei der Verwendung solcher Gemische als Riech- und Aromastoffe.

Das Keton (21) besitzt demgegenüber charakteristische und ausgesprochen positiv zu bewertende Geruchs- und Geschmackseigenschaften, die nicht aufgrund des Vorliegens von (21) in Tabak zu erwarten waren. Die in Referenz-Druckschrift B für (21) angegebenen Aroma-eigenschaften « schwach holzig » beziehen sich lediglich auf den Aromeneffekt aus Tabak. Sie wurden mit nicht näher beschriebenem Tabak vorgenommen ; über die Reinheit des isolierten Naturstoffs (21) wird in dieser Druckschrift nichts gesagt.

Die Anwesenheit von (21) neben den Megastigmatrienonen (3) bis (6) verleiht deartigen Produktgemischen deutlich verbesserte Eigenschaften, wie Tabelle 2 zeigt.

(Siehe Tabelle 2 Seite 7 f.)

Tabelle 2 : Olfaktorische und gustatorische Eigenschaften der Verbindungen (2), (3) bis (6) und (21)

| | Geruch (10 %ig am Riechstreifen) | Geschmack (0.5 ppm in Zuckerwasser) | Geschmack (0.005 % auf Tabak, Typ "american blend") |
|---|---|---|---|
| 2 (aus Beisp. 5) | krautig, grün, etwas fettig | fettig, grün, fruchtig | insgesamt abgeschwächte Tabakeffekte, tabakfremde Grünnote; Nachgeschmack nach Bockshornkleesamen (fenugreek) |
| 3 - 6 (Beisp. 1) | Trockenfrucht, an Heu erinnernd, holzig, Tabakaspekte | schwer fruchtig, balsamisch, Tabakaspekte | mehr Fülle; keine tabakfremde Note, jedoch verminderter "flue cured"-Charakter |
| 21 (Beisp. 4) | weich, krautig, buttrig-milchig (Lacton-artige Note) | weich, fruchtig mit Milch-/Sahne-Aspekte, etwas holzig | vermindertes Zungenbrennen, verstärkte Mundfülle, mehr Körper und verstärkter Inhalationseffekt; verstärkt teerige und heuartige Noten; keine tabakfremden Noten |
| 21 + 3 - 6 (Beisp. 3) | süß-balsamisch, typische Tabaknote, weich-holzig (Sandelholz) Trockenfrucht-/Honig-Aspekte | süß-fruchtig krautig, buttrig, honigartig (gibt Mundfülle, wie warme Milch) | mehr Fülle und Geschmack, Rauch harmonischer, deutliche Süße; der typische "american blend"-Charakter wird verstärkt; keine tabakfremden Noten |

Das bicyclische Keton (21) und Gemische aus dem bicyclischen Keton (21) und den Megastigmatrienonen (3) bis (6) (wobei das Isomere (2) weitgehend vermieden wird) eignen sich daher vorteilhaft als Riechstoffe, bzw. Bestandteile von Parfümölen für kosmetische und technische Verbrauchsgüter sowie als Aromastoffe, bzw. Bestandteile von Aromenmischungen für Nahrungs- und Genußmittel sowie tabak und Tabakerzeugnisse.

Die nachfolgenden Beispiele erläutern die Erfindung, ohne sie zu beschränken. Der Ausdruck « Gaschromatogramm » wird mit « GC » abgekürzt.

Beispiel 1 (Vergleichsbeispiel)

Dehydratisierung von 3-Oxo-α-jonol (1) mit Kalium-hydrogensulfat

a) 10 g 3-Oxo-α-jonol (1) wurden mit 5 g gepulvertem Kaliumhydrogensulfat vermischt und unter Rühren auf 100 °C erhitzt. Nach 8 h Rühren bei 100 °C ließ man auf Raumtemperatur abkühlen und setzte 200 ml Wasser zu. Mehrfache Extraktion mit Benzin, Neutralwaschen der vereinigten organischen Phasen mit Natriumhydrogenkarbonatlösung und Einengen unter vermindertem Druck ergab 8,1 g Produktgemisch ; Gaschromatogramm : Fig. 1.

b) Eine Lösung von 10 g 3-Oxo-α-jonol (1) in 100 ml Toluol wurde mit 5 g Kaliumhydrogensulfat versetzt und 14 h unter Rückfluß zum Sieden erhitzt. Nach Abkühlen auf Raumtemperatur wurde mit Wasser und Natriumhydrogenkarbonatlösung gewaschen und das Lösungsmittel unter vermindertem Druck abdestilliert. Man erhielt 7,9 g Produktgemisch. Gaschromatogramm auf einer GC-Säule der Fa. W. Günther, Düsseldorf (25 m Glaskapillare, stationäre Phase WG 11, Temperaturprogramm : 160-220 °C, 10 °C/min : $t_R$ (min) = 9,1 (26 % (2)), 10,4, 10,8, 11,4, 11,7 (2,1 %, 23,7 %, 3,6 %, 30,4 %, (3) — (6)) ; bei $t_R$ = 10,4 min kein (21).

## Beispiel 2

Dehydratisierung von 3-Oxo-α-jonol (1) mit para-Toluolsulfonsäure

Zu einer am Wasserabscheider siedenten Lösung von 6 g para-Toluolsulfonsäure in 900 ml Toluol wurden innerhalb von 1 h 300 g Oxojonol (1) zugetropft. Nach 1 h Rühren bei Siedetemperatur ließ man auf Raumtemperatur abkühlen und wusch mit Wasser und Natriumkarbonatlösung neutral. Nach Einengen destillierte man über eine 20 cm-Vigreux-Kolonne und erhielt 140 g Produktgemisch (2) — (6) + (21) ; Kp (1 mbar) = 100-120 °C. Gaschromatogramm : Fig. 2.

## Beispiel 3

Eliminierungsreaktion mit 3-Oxo-α-jonol-acetat (22) bei 180-190 °C

Zu einer Lösung von 16 g Natriumacetat (wasserfrei) in 168 g Ethylenglykol wurden unter Rühren bei 180-190 °C innerhalb von 1 h 100 g Oxojonolacetat (22) zugetropft, wobei Essigsäure abdestillierte. Nach 2 h Rühren bei 180-190 °C ließ man abkühlen, versetzte mit Wasser und extrahierte mit Toluol. Trocknen der vereinigten organischen Phasen und Einengen unter vermindertem Druck ergaben 70 g Rohprodukt. Durch Destillation über ein 20 cm-Vigreux-Kolonne wurden 35 g Produktgemisch (2) — (6) + (21) erhalten ; Kp (1 mbar) = 100-140 °C. Gaschromatogramm : Fig. 3.

Eine Lösung von 5 g des obigen Produktgemisches (2) — (6) + (21) in 10 ml Ethylenglykol und 1 g Natriumacetat wurde 16 h bei 200 °C gerührt. Nach üblicher Aufarbeitung lag ein Rohprodukt vor, das zu ca. 70 % (21) (nach GC) enthielt. Das Keton (21) wurde durch Säulenchromatographie an 100 g Kieselgel (Länge 130 cm, Durchmesser 2 cm ; 0,063 — 0,20 mm Korngröße ; Laufmittel Cyclohexan/Ethylacetat = 4 : 1) isoliert ; Reinheit nach GC 95 %, IR-Spektrum : Fig. 5, UV-Spektrum : Fig. 6, $^1$H-NMR-Spektrum : Fig. 7, Massenspektrum : Fig. 8.

## Beispiel 4

Eliminierungsreaktion von 3-Oxojonolacetat (22) bei 200-220 °C

Zu einer Lösung von 24 g Natriumacetat (wasserfrei) in 250 g Ethylenglykol wurden bei Temperaturen von 200-220 °C unter Rühren 150 g Oxojonolacetat (22) innerhalb von 1 h zugetropft, wobei Essigsäure abdestillierte. Nach 2 h Rühren bei 200-220 °C ließ man auf Raumtemperatur abkühlen, versetzte mit Wasser und extrahierte mit Toluol. Nach Neutralwaschen der vereinigten organischen Phasen und Einengen lagen 98 g Rohprodukt vor. Destillation über eine 20 cm-Vigreux-Kolonne ergab 53,5 g bicyclisches Keton (21) (Reinheit nach GC ca. 70 %) ; Kp (0,2 mbar) = 95-125 °C ; Gaschromatogramm : Fig. 4, Ausbeute 32,8 % ; Durch Destillation über eine 80 cm-Metallfüllkörper-Kolonne wurden 35 g (21) in einer Reinheit von 90 % (nach GC) gewonnen.

## Beispiel 5

Pyrolyse von 3-Oxojonolacetat (22)

Bei 500 °C ließ man innerhalb von 2 h 50 g Oxojonolacetat (22) durch ein mit Glasfüllkörpern (Raschigringe, Ø = 5 mm) gefülltes, vertikal gestelltes Glasrohr (Länge 50 cm) laufen, wobei unter leicht vermindertem Druck Essigsäure abdestilliert wurde. Das Reaktionsgut sammelte sich in einem Kolben, Destillation des aufgearbeiteten Rohprodukts über eine 20 cm-Vigreux-Kolonne ergab 26,3 g des Ketons (2), Reinheit nach GC : ca. 80 %. Erneute Destillation über eine 1 m-Drehbandkolonne ergab 15,6 g (2), Kp (1 mbar) = 105 °C. $^1$H-NMR ($CCl_4$) : δ = 0,97 und 1,05, 2 s (1,1—$CH_3$), 1,90, s (5—$CH_3$), 2,57, « s » (6—H), 5,10 und 5,20, 2 m (10, 10—H), 5,56, m (7H), 5,90, « s » (4—H), 6,10, m (8—H), 6,28 ppm, m (9—H).

## Beispiel 6

Parfümbase krautig-holzigen Typs

| | Teile |
|---|---|
| Linalool | 10 |
| Linalylacetat | 30 |
| Lavendelöl, französisch | 20 |
| Bergamottöl, Reggio | 40 |
| 2,2-Dimethyl-3-phenylpropanol (Muguet-alkohol) | 200 |

| | |
|---|---|
| Diheptylacetat | 80 |
| Acetylcedrene (Handelsprodukt « Lignofix ») | 100 |
| Mahagonat | 300 |
| Phyllanton-N | 20 |
| Rosmarinöl, 10 %ig in Dipropylenglykol | 50 |
| Eichenmoos-Extrakt, 10 %ig in Dipropylenglykol | 30 |
| Ysopöl, spanisch | 10 |
| Patchouliöl | 60 |
| | 950 |

a) + 50 Teile (21) (nach Beispiel 4)

b) + 50 Teile (3) — (6) + (21) (nach Beispiel 3)

Diese Parfümbase besitzt einen ausgewogenen Duft krautig-holzigen Typs mit frisch-würzigen Aspekten. Bei Zugabe des Ketons (21) wird der Gesamteindruck weicher, harmonischer und erhält eine natürliche Ausstrahlung, wobei auch blumige Aspekte etwas betont werden.

Bei alternativer Zugabe des Gemisches (3) — (6) + (21) wird der ursprüngliche Geruchseindruck durch eine süß-krautige und eine bitter-grüne Note bereichert und erscheint ebenfalls harmonischer.

Beispiel 7

Erdbeer-Aroma

| | Teile |
|---|---|
| Amylacetat | 34.0 |
| Amylbutyrat | 15.0 |
| Amylvalerianat | 15.0 |
| Anethol | 1.5 |
| Benzylacetat | 85.0 |
| Buttersäure | 15.0 |
| Cinnamylisobutyrat | 7.0 |
| Cinnamylvalerianat | 9.5 |
| Önanthether | 1.5 |
| Diacetyl | 10.0 |
| Ethylacetat | 50.0 |
| Ethylamylketon | 15.0 |
| Ethylbutyrat | 30.0 |
| Ethylcinnamat | 52.0 |
| Ethylheptylat | 2.5 |
| Ethyl-methylphenylglycidat | 260.0 |
| Ethylpropionat | 15.0 |
| Ethylvalerianat | 60.0 |
| Hydroxyphenyl-2-butanon (10 %ig in Ethanol) | 5.0 |
| β-Jonon | 6.5 |
| Citral | 1.0 |
| Maltol | 70.0 |
| Methylanthranilat | 7.0 |
| Methylcinnamat | 35.5 |
| Methylsalicylat | 6.5 |
| Neroliöl | 0.5 |
| Phenylethylalkohol | 1.5 |
| γ-Undecalacton | 58.5 |
| Vanillin | 70.0 |
| Triethylcitrat | 1 000.0 |
| Ethanol | 60.0 |
| | 2 000.0 |

Dieses Aromakonzentrat besitzt bei üblicher Dosierung ein kräftiges Aroma vom Erdbeer-Typ. Werden anstelle des Ethanols 60 Teile des Ketons (21) (nach Beispiel 4) eingearbeitet, so besitzt das resultierende Aroma mehr weiche, cremige Geschmacksaspekte, die dem Eindruck des natürlichen Erdbeeraromas näherkommen. Bei alternativem Einsatz des Produktgemisches (21) + (3) — (6) (nach Beispiel 3) erhält man ebenfalls eine positive Modifikation des ursprünglichen Erdbeeraromas, da nunmehr mehr Fülle, aber auch holzige Aspekte auftreten, die an Erdbeerkerne erinnern.

(Siehe Tabelle Seite 10 f.)

Beispiel 8

TABAKAROMA "VIRGINIA-ENHANCER"

| | | Teile |
|---|---|---|
| Angelikawurzelöl | (1% in Propylenglykol) | 5,- |
| Kardamomenol | (1% in Propylenglykol) | 20,- |
| Bayöl | (1% in Ethanol) | 10,- |
| Bockshornkleesamen-Extrakt | | 2,- |
| Liebstocköl | | 0,5 |
| Furfural | | 5,- |
| Cumarin | | 15,- |
| Vanille-Extrakt | (10% in Propylenglykol) | 150,- |
| Nelkenblätteröl | | 5,- |
| Corianderöl | | 1,- |
| Amylisovalerianat | | 5, |
| Ethanol | | 201,5 |
| Propylenglykol | | 550,- |
| | | --------- |
| | | 970,- |

Diese Mischung bewirkt bei einer Dosierung von 80 g/100 kg Tabak eine Verstärkung der dunklen Tabaknoten und im Rauch mehr Mundfülle und eine Betonnung würzig-teeriger Aspekte. Bei Zugabe von 30 Teilen des Gemischs (2) — (6) + (21) (nach Beispiel 3) liegt ein Aroma vor, das bei gleicher Dosierung eine deutlich stärkere Tabaknote vom Typ Virginia aufweist.

**Patentansprüche**

1. Verfahren zur Herstellung von 4,4,7-Trimethyl-3,4,7,8-tetrahydro-2 (6H)-naphthalin-on der Formel

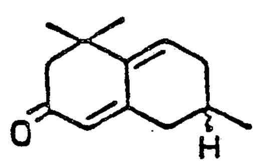

in reiner Form oder im Gemisch mit 3,5,5-Trimethyl-4-butenyliden-cyclo-hex-2-en-1-onen der allgemeinen Formel

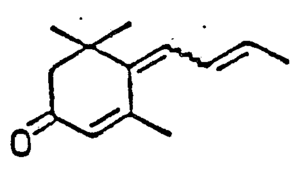

dadurch gekennzeichnet, daß man 3-Oxo-α-jonol der Formel 1

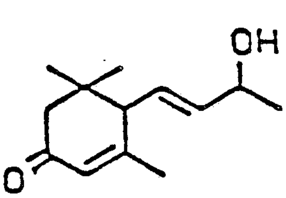

(I)

einer Eliminierungsreaktion unterwirft entweder

a) durch Zugabe des 3-Oxo-α-jonols zu einer Lösung einer protischen Säure in einem inerten Lösungsmittel bei erhöhter Temperatur und Abscheiden des Reaktionswassers in üblicher bekannter Weise oder

b) durch Überführung des 3-Oxo-α-jonols in dessen Niedrigalkylester und Behandeln desselben mit Alkali- oder Erdalkalihydroxiden oder -acetaten in einem aprotischen Lösungsmittel bei Temperaturen von 150-300 °C,

und gegebenenfalls aus dem erhaltenen Gemisch das 4,4,7-Trimethyl-3,4,7,8-tetrahydro-2(6H)-naphthalin-on zu dessen Anreicherung oder Reindarstellung abtrennt, wobei man gegebenenfalls die neben 4,4,7-Trimethyl-3,4,7,8-Tetrahydro-2(6H)-naphthalin-on entstehenden 3,5,5-Trimethyl-4-butenyliden-cyclohex-2-en-1-one — vor oder nach Entfernung des Naphthalin-ons — durch Verlängern der Reaktion unter analogen Reaktionsbedingungen gemäß Variante (b) zu weiterem 4,4,7-Trimethyl-3,4,7,8-tetrahydro-2(6H)-naphthalin-on cyclisiert.

2. Verfahren nach Anspruch 1, dadurch gekennzeichnet, daß man als protische Säure eine Arylsulfonsäure und als inertes Lösungsmittel einen aromatischen Kohlenwasserstoff einsetzt.

3. Verfahren nach den Ansprüchen 1 und 2, dadurch gekennzeichnet, daß man das 3-Oxo-α-jonol zu einer Lösung von p-Toluolsulfonsäure in Toluol, vorzugsweise bei deren Siedetemperatur, zugibt.

4. Verfahren nach Anspruch 1, dadurch gekennzeichnet, daß man das 3-Oxo-α-jonol in dessen Acetat überführt und dieses mit Natriumacetat behandelt.

5. Verfahren nach Anspruch 4, dadurch gekennzeichnet, daß man das 3-Oxo-α-jonol-acetat mit Natriumacetat in Ethylenglycol behandelt.

6. Verfahren nach Ansprüchen 1, 4 oder 5, dadurch gekennzeichnet, daß man den 3-Oxo-α-jonol-niedrigalkylester bei Temperaturen von 180 bis 230 °C behandelt.

7. Verfahren nach Anspruch 1, dadurch gekennzeichnet, daß man das 4,4,7-Trimethyl-3,4,7,8-tetrahydro-2(6H)-naphthalin-on durch Destillation oder chromatographische Methoden abtrennt.

8. Verfahren zur Parfümierung kosmetischer und technischer Verbrauchsgüter und zur Aromatisierung von Nahrungs- und Genußmittel sowie Tabak, dadurch gekennzeichnet, daß das nach dem Verfahren gemäß Anspruch 1 hergestellte 4,4,7-Trimethyl-3,4,7,8-tetrahydro-2(6H)-naphthalin-on als Riechstoff oder Aromastoff in reiner Form oder im Gemisch mit den gebildeten 3,3,5-Trimethyl-4-butenyliden-cyclohex-2-en-1-onen verwendet wird.

## Claims

1. A process for the production of 4,4,7-trimethyl-3,4,7,8-tetrahydro-2(6H)-naphthalene-one of the formula

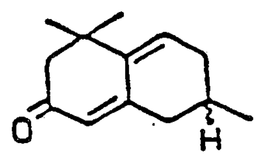

in pure form or as mixed with 3,5,5-trimethyl-4-butenylidene-cyclo-hex-2-en-1-ones of the general Formula

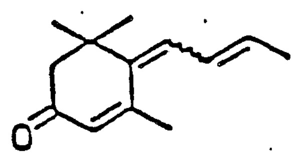

characterized in that 3-oxo-alpha-ionol of the formula 1

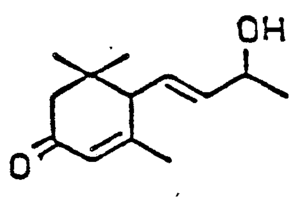

(I)

is subjected to an elimination reaction either

a) by adding of the 3-oxo-alpha-ionol to a solution of a protic acid in an inert solvent at elevated temperature and separation of the water of reaction in a conventional way, or

b) by converting of the 3-oxo-alpha-ionol into its low-carbon alkyl ester and treating the latter with

hydroxides or acetates of alkali metals or alkaline-earth metals in a non-protic solvent at temperatures of 150 to 300 °C,
and, as the case may be, the 4,4,7-trimethyl-3,4,7,8-tetrahydro-2(6H)-naphthalene-one is separated from the resulting mixture for enrichment or rectification, wherein, as the case may be, the 3,5,5-trimethyl-4-butenylidene-cyclo-hex-2-en-1-ones which are formed together with 4,4,7-trimethyl-3,4,7,8-tetrahydro-2(6H)-naphthalene-one are cyclized, before or after the separation of the naphthalene-one, by extending the reaction under analogous conditions as used according to variant (b) to yield further amounts of the 4,4,7-trimethyl-3,4,7,8-tetrahydro-2(6H)-naphthalene-one.

2. A process according to claim 1 characterized in that an arylsulfonic is used as said protic acid and an aromatic hydrocarbon is used as said inert solvent.

3. A process according to claims 1 and 2, characterized in that the 3-oxo-alpha-ionol is added to a solution of p-toluene-sulfonic acid in toluene, preferably at the boiling point of the solution.

4. A process according to claim 1, characterized in that the 3-oxo-alpha-ionol is converted into its acetate which is then treated with sodium acetate.

5. A process according to claim 4, characterized in that the 3-oxo-alpha-ionol acetate is treated with sodium acetate in ethylene glycol.

6. A process according to claim 1, 4 oder 5, characterized in that the 3-oxo-alpha-ionol low-carbon alkyl ester is treated at temperatures from 180 to 230 °C.

7. A process according to claim 1, characterized in that the 4,4,7-trimethyl-3,4,7,8-tetrahydro-2(6H)-naphthalene-one is separated by means of distillation or chromatographic methods.

8. A process for scenting cosmetic and technical goods and for aromatizing foods and semi-luxuries as well as tobacco, characterized in that the 4,4,7-trimethyl-3,4,7,8-tetrahydro-2(6H)-naphthalene-one prepared according to claim 1 is used as odiferous or fragrant substance in pure form or admixed with the 3,3,5-trimethyl-4-butenylidene-cyclohex-2-en-1-ones which have been formed.

**Revendications**

1. Procédé pour la préparation de la 4,4,7-triméthyl-3,4,7,8-tétrahydro-2(6H)-naphtalénone de formule

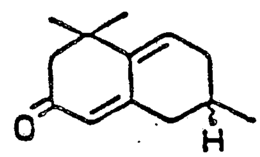

sous forme pure ou en mélange avec des 3,5,5-triméthyl-4-buténylidène-cyclohex-2-ène-1-ones de formule générale

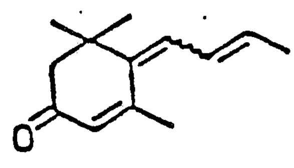

caractérisé en ce qu'on soumet du 3-oxo-α-ionol de formule 1

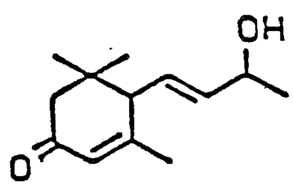

(I)

à une réaction d'élimination,

a) soit par addition du 3-oxo-α-ionol à une solution d'un acide protique dans un solvant inerte à haute température, et séparation de l'eau de réaction d'une manière connue en soi,

b) soit par conversion du 3-oxo-α-ionol en son ester alkylique inférieur, et traitement de ce dernier avec des hydroxydes ou des acétates de métaux alcalins ou alcalinoterreux, dans un solvant aprotique, à des températures de 150 à 300 °C,

et que, éventuellement, on sépare du mélange obtenu la 4,4,7-triméthyl-3,4,7,8-tétrahydro-2(6H)-naphtalénone, pour son enrichissement ou sa préparation à l'état de pureté, ce à l'occasion de quoi on cyclise éventuellement les 3,5,5-triméthyl-4-buténylidène-cyclohex-2-ène-1-ones obtenues en plus de la 4,4,7-triméthyl-3,4,7,8-tétrahydro-2(6H)-naphtalénone, avant ou après élimination de la naphtalénone, par

prolongement de la réaction dans des conditions de réaction analogues selon la variante (b), pour obtenir une quantité supplémentaire de 4,4,7-triméthyl-3,4,7,8-tétrahydro-2(6H)-naphtalénone.

2. Procédé selon la revendication 1, caractérisé en ce qu'on utilise comme acide protique un acide arylsulfonique, et comme solvant inerte un hydrocarbure aromatique.

3. Procédé selon les revendications 1 et 2, caractérisé en ce qu'on ajoute le 3-oxo-α-ionol à une solution d'acide p-toluènesulfonique dans du toluène, de préférence à sa température d'ébullition.

4. Procédé selon la revendication 1, caractérisé en ce qu'on convertit le 3-oxo-α-ionol en son acétate, et qu'on traite ce dernier avec de l'acétate de sodium.

5. Procédé selon la revendication 4, caractérisé en ce qu'on traite l'acétate de 3-oxo-α-ionol avec de l'acétate de sodium dans de l'éthylèneglycol.

6. Procédé selon les revendications 1, 4 ou 5, caractérisé en ce qu'on traite l'ester alkylique inférieur du 3-oxo-α-ionol à des températures de 180 à 230 °C.

7. Procédé selon la revendication 1, caractérisé en ce qu'on sépare la 4,4,7-triméthyl-3,4,7,8-tétrahydro-2(6H)-naphthalénone par distillation ou par des méthodes chromatographiques.

8. Procédé pour le parfumage de produits de consommation de nature cosmétique et technique, et pour aromatiser des produits alimentaires et des produits de consommation de luxe tels le tabac, caractérisé en ce qu'on utlise la 4,4,7-triméthyl-3,4,7,8-tétrahydro-2(6H)-naphtalénone préparée par le procédé selon la revendication 1, en tant que matière odorante ou aromatisante, sous forme pure ou en mélange avec les 3,3,5-triméthyl-4-buténylidène-cyclohex-2-ène-1-ones formées.

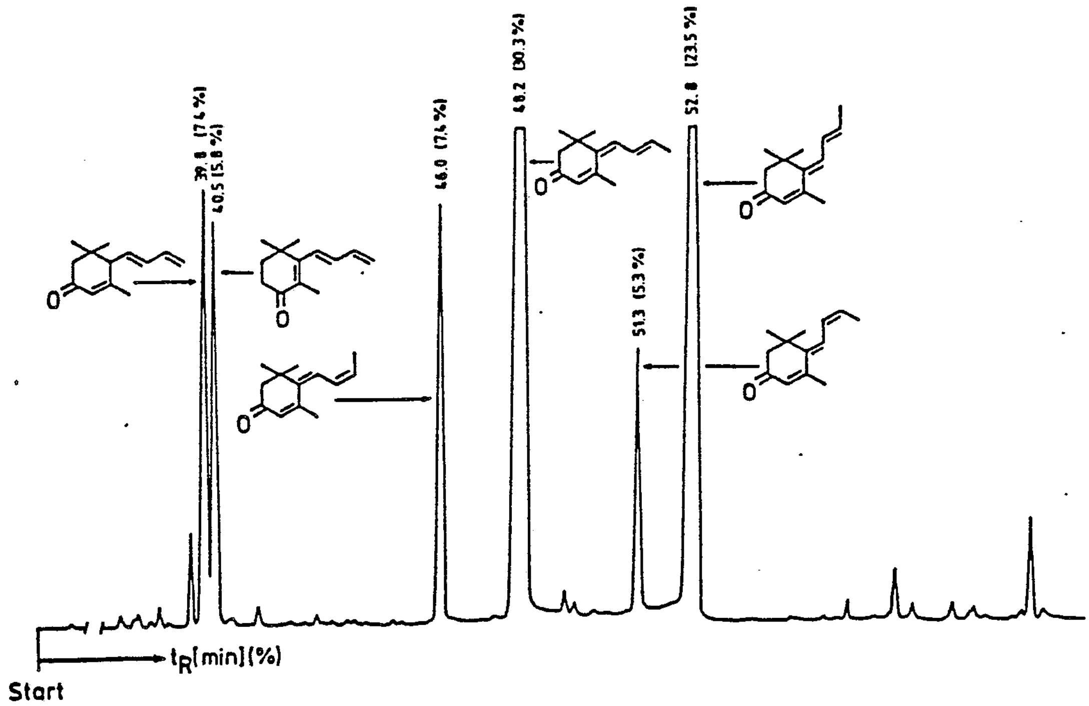


Fig. 1: Gaschromatogramm (60 m Glaskapillare, DB Wax, Temperaturprogramm 100-200°C, 2°C/min) des Dehydratisierungsproduktgemisches von 3-Oxo-α-jonol nach Beispiel 1 ($KHSO_4$/100°C).

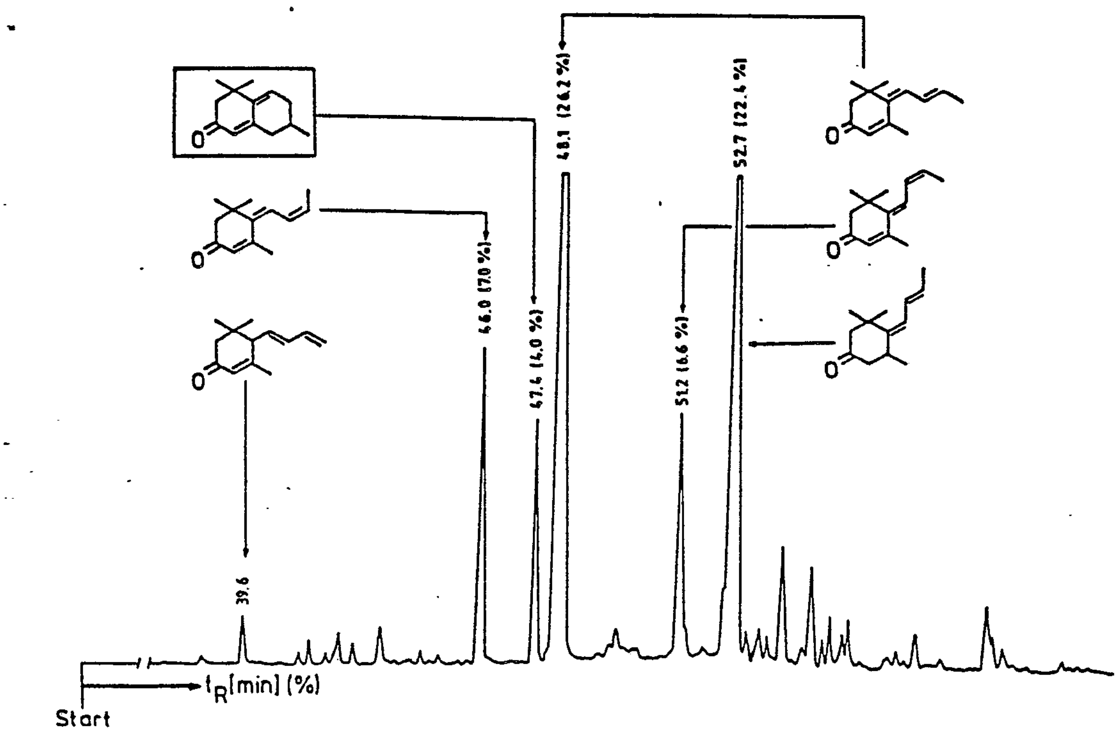


Fig. 2: Gaschromatogramm (60 m Glaskapillare, DB-Wax, Temperaturprogramm 100-200°C, 2°C/min) des Dehydratisierungsgemisches von 3-Oxo-α-jonol nach Beispiel 2

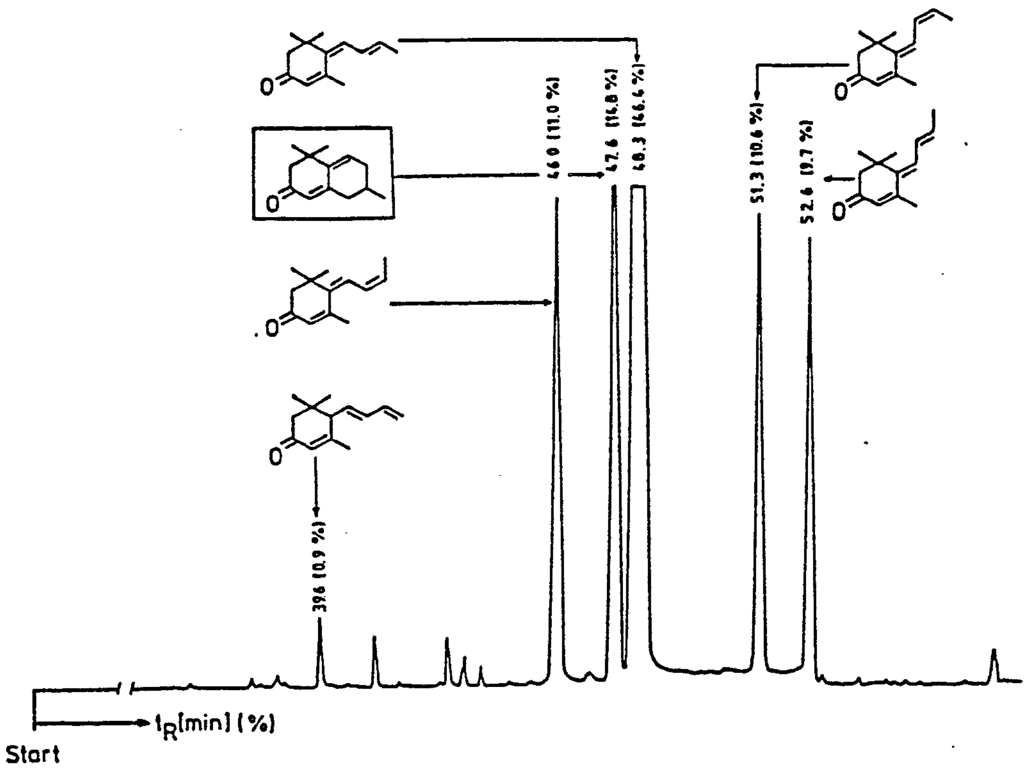


Fig. 3: Gaschromatogramm (60 m Glaskapillare, DB Wax, Temperaturprogramm 100-200°C, 2°C/min) des Produktgemisches nach Beispiel 3

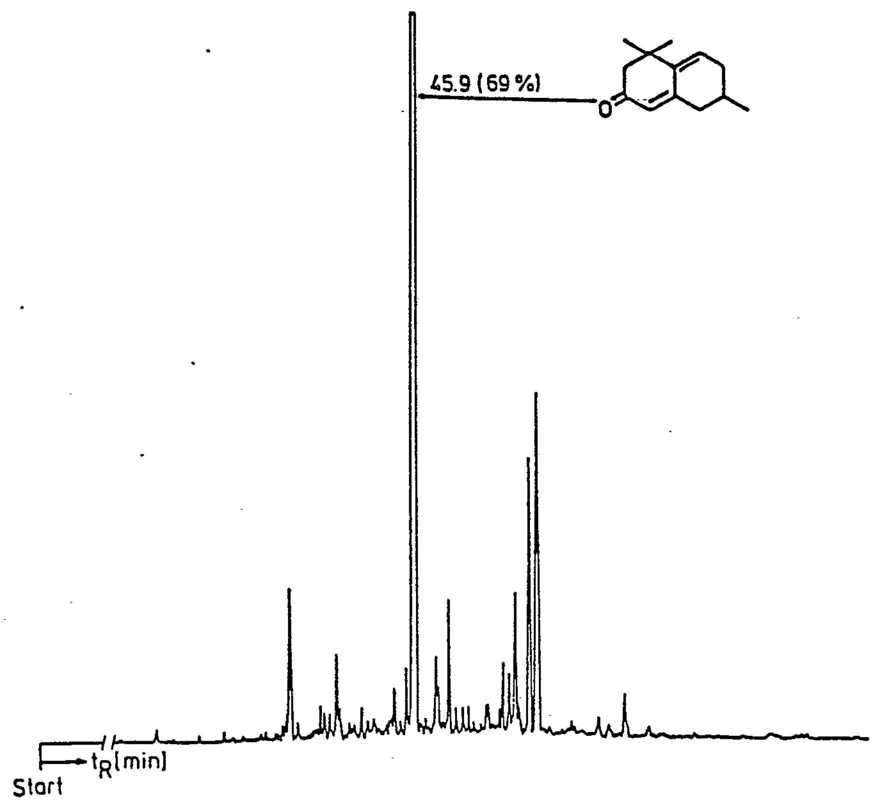


Fig. 4: Gaschromatogramm (30 m Glaskapillare, DB Wax, Temperaturprogramm 100-240°C, 10°C/min) des Produktgemisches nach Beispiel 4

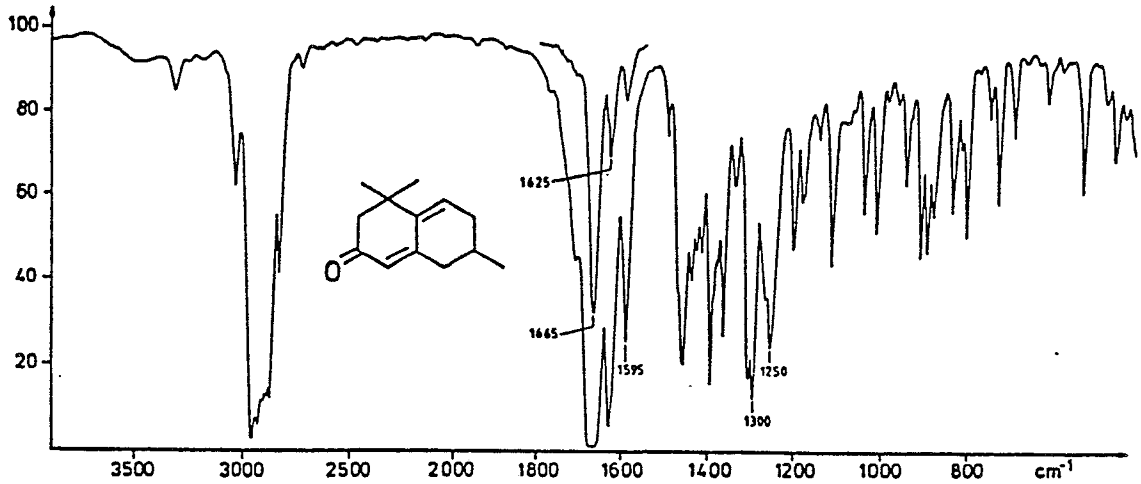


Fig. 5: IR-Spektrum (Film) von 21

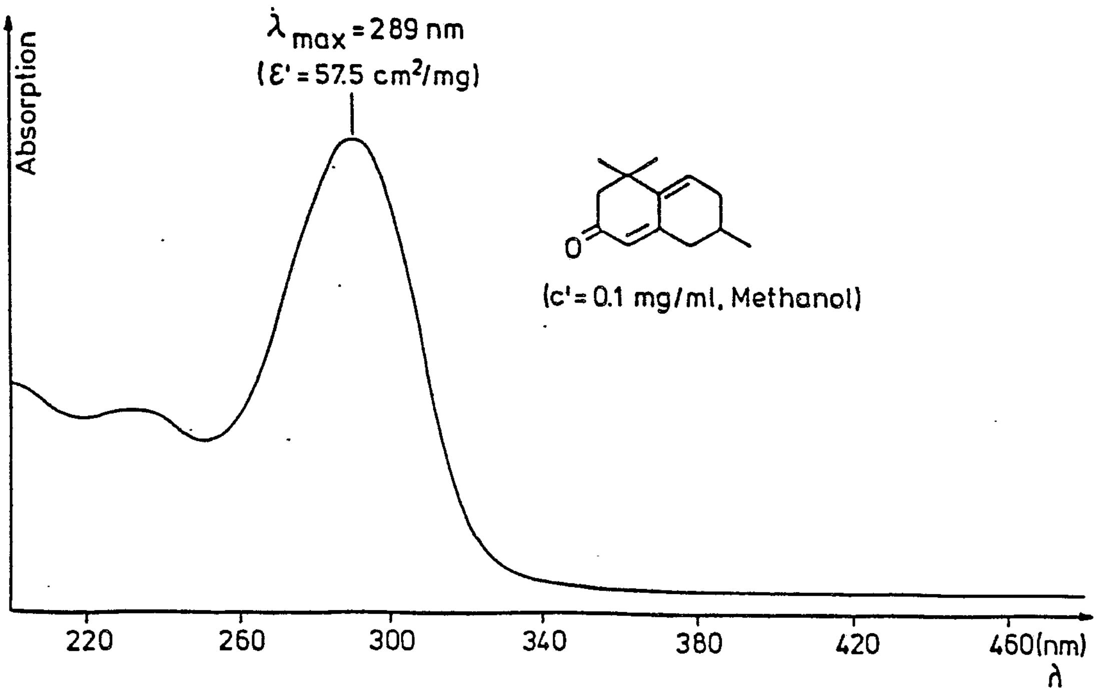


Fig. 6: UV-Spektrum (Methanol) von 21

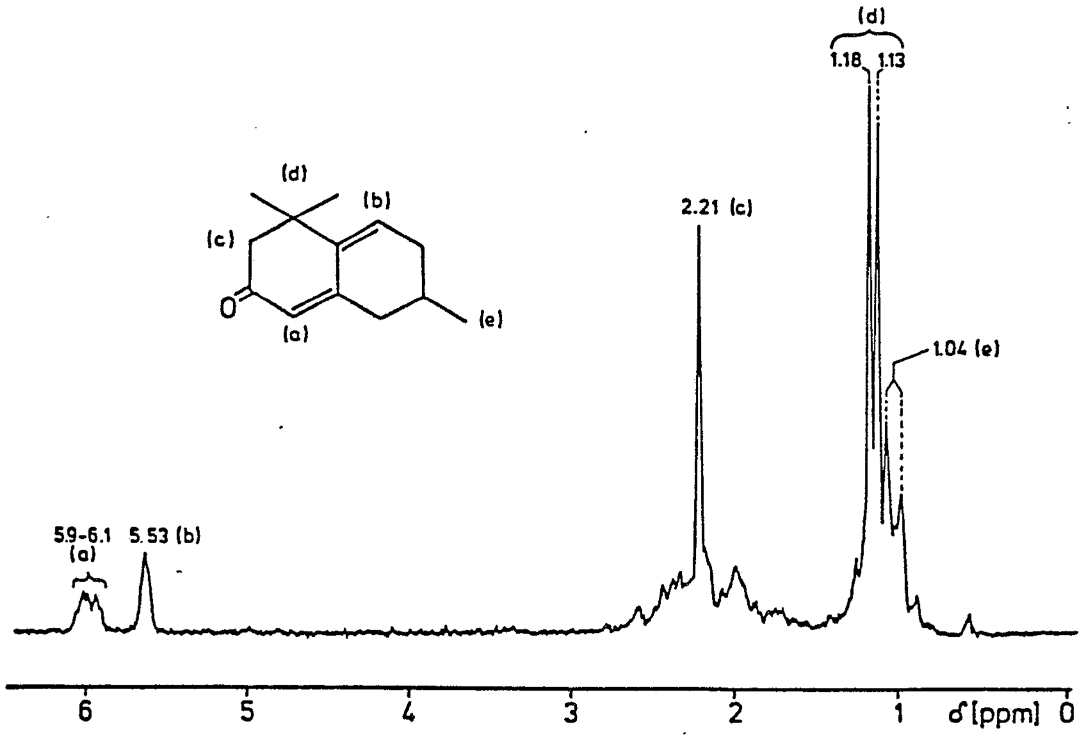


Fig. 7: $^1$H-NMR-Spektrum (60 mHz, $CCl_4$, TMS als innerer Standard) von 21

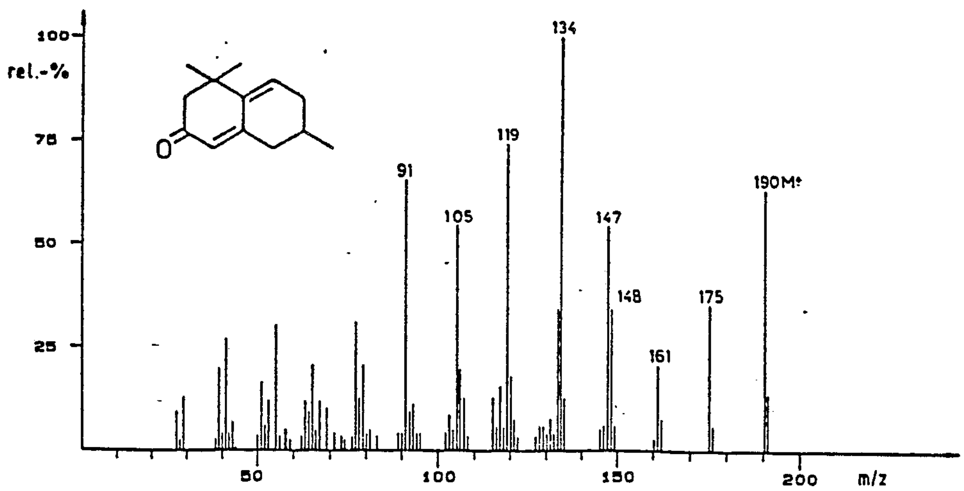


Fig. 8: Massenspektrum (80 eV) von 21